# EUROPÄISCHE PATENTSCHRIFT

(11) **EP 1 205 469 B1**
(45) Veröffentlichungstag und Bekanntmachung des Hinweises auf die Patenterteilung: **04.01.2006**
(21) Anmeldenummer: 01125351.5
(22) Anmeldetag: 29.10.2001
(51) Int. Cl.: C07C 209/38, C07C 211/55

(54) **Verfahren zur Herstellung von 4-Aminodiphenylamin**
Process for the preparation of 4-amino diphenylamine
Procédé de préparation de 4-amino diphénylamine

(30) Priorität: 08.11.2000 DE 10055221
(43) Veröffentlichungstag der Anmeldung: 15.05.2002
(73) Patentinhaber: LANXESS Deutschland GmbH, 51369 Leverkusen (DE)
(72) Erfinder: Schelhaas, Michael, Dr., 50733 Köln (DE); Casser, Carl, Dr., 13585 Berlin (DE); Bielefeldt, Dietmar, Dr., 40883 Ratingen (DE); Ooms, Pieter, Dr., 47800 Krefeld (DE); Haider, Joachim, Dr., 50674 Köln (DE); Jautelat, Manfred, Dr., 51399 Burscheid (DE); Laue, Christian, Dr., 40789 Monheim (DE); Giera, Henry, Dr., 86862 Grosskitzingen (DE)

(56) Entgegenhaltungen:
- EP-A- 0 895 983
- EP-A- 0 941 985
- US-A- 4 404 401

## Beschreibung

Die Erfindung betrifft ein Verfahren zur Herstellung von 4-Aminodiphenylamin (4-ADPA), einem wichtigen Einsatzprodukt zur Synthese von Alterungsschutzmitteln und Stabilisatoren in der Gummi- und Polymerindustrie (Kirk-Othmer, Encyclopedia of Chemical Technology, 4^{th} Edition, 1992, Vol. A3, Seite 424-456; Ullmann's Ecyclopedia of Industrial Chemistry, 5^{th} Edition, Vol. A3, 1985, Seite 91-111).

4-Aminodiphenylamin kann nach verschiedenen Methoden hergestellt werden. Eine Möglichkeit ist die zweistufige (Zwischenprodukt 4-Nitrodiphenylamin) Umsetzung von Anilin bzw. Anilinderivaten mit p-Chlomitrobenzol in Gegenwart eines Säureakzeptors oder eines Neutralisierungsmittels und gegebenenfalls in Gegenwart eines Katalysators. Die Herstellung nach dieser Methode ist beispielsweise in DE-A 3 501 698, DE-A 1 856 63, US-A 4 670 595, US-A 4 187 249, US-A 468 333 und US-A 4 187 248 beschrieben. Ein Nachteil dieses Verfahrens ist, dass die anfallenden Halogenidionen mit beträchtlichen Kosten entsorgt werden müssen und die Ausgangsmaterialien, wie p-Chlornitrobenzol bzw. die entsprechenden Formanilidderivate, in zusätzlichen Reaktionsschritten hergestellt werden müssen.

Eine weitere Möglichkeit zur Herstellung von 4-ADPA besteht in der Umsetzung von Anilin bzw. entsprechenden Anilinderivaten mit Nitrobenzol in Gegenwart von Tetraalkylammoniumhydroxiden und in Gegenwart kontrollierter Mengen an protischen Materialien (siehe WO 93/00324 und WO 93/24450). Nachteilig hierbei ist die geringe thermische Beständigkeit der Tetraalkylammoniumhydroxide, so dass diese nicht vollständig in den Prozess zurückgeführt werden können.

Die einstufige Herstellung von 4-ADPA aus Nitrobenzol bzw. Nitrosobenzol in Gegenwart von Wasserstoff, eines Hydrierkatalysators und in Gegenwart von Basen liefert nur unbefriedigende Ausbeuten an 4-ADPA (siehe DE-A 19 70 91 24 und DE-A 19 734 055).

Eine weitere Möglichkeit zur Herstellung von 4-ADPA besteht in der säurekatalysierten Dimerisierung von Nitrosobenzol zu 4-Nitrosophenyl-phenylhydroxylamin und anschließender Reduktion zum 4-ADPA (siehe DE-A 1 147 237 und DE-A 2 703 919). Nachteil dieser Verfahren ist die notwendige Isolierung des thermisch instabilen 4-Nitrosophenyl-phenylhydroxylamins und das hierbei gegebenenfalls durch Neutralisation anfallende Abwasser.

Gegenstand der vorliegenden Erfindung ist ein Verfahren zur Herstellung von 4-ADPA, dass dadurch gekennzeichnet ist, dass Nitrosobenzol in Gegenwart einer Protonensäure als Katalysator und in Gegenwart eines Hydrierkatalysators, gegebenenfalls in Gegenwart eines inerten organischen Lösungsmittels, mit Wasserstoff hydriert, den Hydrierkatalysator aus dem Reaktionsgemisch abtrennt, die Protonensäure und das gegebenenfalls eingesetzte Lösungsmittel abdestilliert und das hierbei anfallende 4-ADPA-Ammoniumsalz thermisch gespalten wird, wobei 4-ADPA entsteht.

Als für das erfindungsgemäße Verfahren geeignete Protonensäure kommen in Frage aliphatische und aromatische Sulfonsäuren wie Methansulfonsäuren und Benzolsulfonsäuren, Fluorwasserstoff und Trifluoressigsäure. Mit steigendem Wassergehalt der erfindungsgemäßen Säuren sinkt in der Regel die Ausbeute an 4-ADPA, so dass der Einsatz wasserfreier Säuren bevorzugt ist. Erfindungsgemäß werden die Säuren in Mengen von 0,1 bis 100, bevorzugt 1 bis 100 Mol Nitrosobenzol eingesetzt. Die Säuren können sowohl einzeln als auch im Gemisch untereinander eingesetzt werden.

Als Hydrierkatalysatoren eignen sich für das erfindungsgemäße Verfahren praktisch alle heterogenen Katalysatoren, die als Hydrierkatalysatoren bekannt sind. Die erfindungsgemäßen Katalysatoren umfassen Metalle der 8.-10. Gruppe des Periodensystems (nach IUPAC, neu) oder Kupfer und/oder Chrom auf geeignetem Träger mit einem Metallgehalt von 0,01 bis 50 Gew.-%, bevorzugt 0,1 bis 20 Gew.-%, bezogen auf das Gesamtgewicht des Katalysators. Erfindungsgemäß können Katalysatoren eingesetzt werden, die eines oder mehrere der oben genannten Metalle enthalten. Bevorzugte Metalle sind insbesondere Platin, Palladium und Rhodium, besonders bevorzugt sind Platin und Palladium. Weitere bevorzugte Katalysatoren sind Raney-Nickel und geträgerte Nickelkatalysatoren. Erfindungsgemäß können auch die obengenannten Metalle oder ihre Verbindungen in reiner Form als Feststoff eingesetzt werden. Als Beispiele für ein Metall in reiner Form seien Palladium- und Platinschwarz genannt.

Die erfindungsgemäßen Katalysatoren können in diskontinuierlichen Verfahrensvarianten in Mengen von 0,01 bis 20 Gew.-%, bezogen auf eingesetztes Nitrosobenzol verwendet werden, bevorzugt in Mengen von 0,01 bis 10 Gew.-%. Bei kontinuierlicher Durchführung der Reaktion, beispielsweise in einem Rührkessel mit pulverförmigen Katalysator oder in der Rieselphase am Festbettkatalysator, können Belastungen von 0,01 bis 500 g Nitrosobenzol pro g Katalysator und Stunde verwendet werden.

Die Reaktionstemperaturen bei dem erfindungsgemäßen Verfahren betragen - 20°C bis 50°C, insbesondere -10°C bis 30°C; der Wasserstoffdruck liegt bei 0,1 bis 150 bar, insbesondere bei 0,5 bis 70 bar, ganz besonders bevorzugt bei 1 bis 50 bar.

Das erfindungsgemäße Verfahren kann auch in Gegenwart von organischen Lösungsmitteln durchgeführt werden. Besonders bevorzugt sind aprotische Lösungsmittel, die gegenüber der verwendeten Protonensäure und unter den Hydrierbedingungen inert sind. Als inerte organische aprotische Lösungsmittel kommen aliphatische oder aromatische Kohlenwasserstoffe, lineare oder cyclische Ether, halogenierte aliphatische oder aromatische Kohlenwasserstoffe oder deren Gemische in Betracht. Als geeignete Lösungsmittel seien insbesondere benannt: Benzol, Toluol, Xylol, tert.-Butylmethylether, Dioxan, Tetrahydrofuran, Chloroform, Methylenchlorid und/oder Chlorbenzol. Die Menge an Lösungsmittel ist für das erfindungsgemäße Verfahren nicht kritisch. Die geeignete Menge kann ebenfalls leicht durch entsprechende Vorversuche ermittelt werden. Bei einer kontinuierlichen Dosierung von Nitrosobenzol und Katalysatorsäure hängt die Menge an verwendetem Lösungsmittel insbesondere von der Löslichkeit des Nitrosobenzols im verwendeten Lösungsmittel ab.

Die Durchführung des erfindungsgemäßen Verfahrens kann sowohl kontinuierlich als auch diskontinuierlich erfolgen. Bei der diskontinuierlichen Fahrweise wird Nitrosobenzol gegebenenfalls in Anwesenheit eines Lösungsmittels mit der Protonensäure versetzt und das erhaltene Reaktionsgemisch anschließend in Gegenwart eines Hydrierkatalysators mit Wasserstoff hydriert. Kontinuierliche Verfahrensvarianten können in den dem Fachmann bekannten Apparaturen zur Kontaktierung von Fest-, Flüssig- und Gasphasen durchgeführt werden. Insbesondere kommen hier Rührkessel, Umpumpreaktoren, Busreaktoren, im Gleich- oder Gegenstrom betriebene Blasensäulen oder Rieselphasenreaktoren oder Kaskaden dieser Reaktoren in Frage.

Die erfindungsgemäße Aufarbeitung des Reaktionsgemisches besteht darin, dass man das Reaktionsgemisch nach Filtration des Hydrierkatalysators destillativ aufarbeitet. Hierbei lässt sich die eingesetzte Protonensäure sowie gegebenenfalls das eingesetzte Lösungsmittel praktisch quantitativ zurückgewinnen. Bei dem erfindungsgemäßen Verfahren entsteht kein Abwasser, was ökonomisch und ökologisch von großem Vorteil ist.

Das verbleibende 4-ADPA-Salz der entsprechenden Protonensäuren wird zur Gewinnung von 4-ADPA thermisch bei Temperaturen von ca. 50 bis 200°C, und Drücken von 1013 bis 0,05 mbar gespalten.

### Beispiele

### Beispiel 1

In einem Autoklaven werden bei 0°C zu 21,4 g Nitrosobenzol und 1 g Pd/C (5 %ig) 100 ml wasserfreie Flusssäure gegeben. Das Reaktionsgemisch wird auf 10°C erwärmt und anschließend bei 30 bar Wasserstoffdruck hydriert. Nach Filtration des Hydrierkatalysators wird die Katalysatorsäure (Flusssäure) abdestilliert. Das verbleibende 4-ADPA-Ammoniumsalz wird thermisch bei 200°C und 16 mbar gespalten, wobei 17 g 4-ADPA mit einem Fluoridgehalt < 0,1 % erhalten werden.

### Beispiel 2

Analog Beispiel 1 werden als Katalysatorsäure 100 ml Trifluoressigsäure verwendet. Das hierbei erhaltene 4-ADPA-Ammoniumsalz wird bei 100°C und 1 mbar thermisch gespalten, wobei 15 g 4-ADPA erhalten werden.

## Patentansprüche

1. Verfahren zur Herstellung von 4-Aminodiphenylamin (4-ADPA), **dadurch gekennzeichnet, dass** man Nitrosobenzol in Gegenwart einer Protonensäure als Katalysator und in Gegenwart eines Hydrierkatalysators, gegebenenfalls in Gegenwart eines inerten organischen Lösungsmittels, mit Wasserstoff hydriert, den Hydrierkatalysator aus dem Reaktionsgemisch abtrennt, die Protonensäure und das gegebenenfalls eingesetzte Lösungsmittel abdestilliert und das hierbei anfallende 4-ADPA-Ammoniumsalz thermisch spaltet, wobei 4-ADPA erhalten wird.

2. Verfahren nach Anspruch 1, **dadurch gekennzeichnet**, das man die Hydrierung bei Temperaturen von -20 bis 50°C und Drücken von 0,1 bis 150 bar durchführt.

3. Verfahren nach Anspruch 1, **dadurch gekennzeichnet, dass** man die thermische Spaltung des 4-ADPA-Ammoniumsalzes bei Temperaturen von 50 bis 200°C und Drücken von 1013 bis 0,05 mbar durchführt.

4. Verfahren nach Anspruch 1, **dadurch gekennzeichnet, dass** man als Protonensäure aliphatische und aromatische Sulfonsäuren, Fluorwasserstoff und/oder Trifluoressigsäure einsetzt.

5. Verfahren nach Anspruch 1, **dadurch gekennzeichnet, dass** man als organische Lösungsmittel aliphatische oder aromatische Kohlenwasserstoffe, lineare oder cyclische Ether, halogenierte aliphatische oder aromatische Kohlenwasserstoffe oder deren Gemische einsetzt.

## Claims

1. A process for preparing 4-aminodiphenylamine (4-ADPA), **characterised in that** nitrosobenzene is hydrogenated with hydrogen in the presence of a proton acid as catalyst and in the presence of a hydrogenation catalyst, optionally in the presence of an inert organic solvent, the hydrogenation catalyst is separated off from the reaction mixture, the proton acid and the optionally used solvent are distilled off and the 4-ADPA ammonium salt obtained in this way is thermally decomposed, wherein 4-ADPA is obtained.

2. A process according to claim 1, **characterised in that** hydrogenation is performed at temperatures of -20°C to 50°C and pressures of 0.1 to 150 bar.

3. A process according to claim 1, **characterised in that** thermal decomposition of the 4-ADPA ammonium salt is performed at temperatures of 50 to 200°C and pressures of 1013 to 0.05 mbar.

4. A process according to claim 1, **characterised in that** aliphatic and aromatic sulfonic acids, hydrogen fluoride and/or trifluoroacetic acid are used as proton acids.

5. A process according to claim 1, **characterised in that** aliphatic or aromatic hydrocarbons, linear or cyclic ethers, halogenated aliphatic or aromatic hydrocarbons or their mixtures are used as organic solvents.

## Revendications

1. Procédé pour la préparation de la 4-aminodiphénylamine (4-ADPA), **caractérisé en ce que** l'on hydrogène le nitrosobenzène en présence d'un acide protonique qui sert de catalyseur et en présence d'un catalyseur d'hydrogénation, le cas échéant en présence d'un solvant organique inerte, on sépare le catalyseur d'hydrogénation du mélange de réaction, on distille l'acide protonique et le solvant éventuellement utilisé, ce qui donne le sel de 4-ADPA-ammonium d'où l'on tire la 4-ADPA par scission à la chaleur.

2. Procédé selon revendication 1, **caractérisé en ce que** l'hydrogénation est réalisée à des températures de -20 à +50°C sous des pressions de 0,1 à 150 bar.

3. Procédé selon revendication 1, **caractérisé en ce que** la scission à la chaleur du sel de 4-ADPA-ammonium est réalisée à des températures de 50 à 200°C sous des pressions de 1 013 à 0,05 mbar.

4. Procédé selon revendication 1, **caractérisé en ce que** l'acide protonique utilisé est un acide sulfonique aliphatique ou aromatique, le fluorure d'hydrogène et/ou l'acide trifluoracétique.

5. Procédé selon revendication 1, **caractérisé en ce que** le solvant organique utilisé est choisi parmi les hydrocarbures aliphatiques ou aromatiques, les éthers linéaires ou cycliques, les hydrocarbures aliphatiques ou aromatiques halogénés ou leurs mélanges.
